# EUROPEAN PATENT SPECIFICATION

(11) **EP 4 039 214 B1**
(45) Date of publication and mention of the grant of the patent: **03.12.2025**
(21) Application number: 22155139.3
(22) Date of filing: 04.02.2022
(51) Int. Cl.: A61B 18/14, A61M 25/10, A61B 34/20, A61B 34/32, A61B 17/00, A61B 34/30, A61B 18/00, A61B 90/00

(54) **SYSTEM FOR ALIGNMENT OF BALLOON ABLATION CATHETER IN PULMONARY VEIN**
SYSTEM ZUR AUSRICHTUNG EINES BALLONABLATIONSKATHETERS IN EINER PULMONALVENE
SYSTÈME D'ALIGNEMENT DE CATHÉTER D'ABLATION À BALLONNET DANS UNE VEINE PULMONAIRE

(30) Priority: 05.02.2021 US 202117169183
(43) Date of publication of application: 10.08.2022
(73) Proprietor: Biosense Webster (Israel) Ltd., Yokneam 2066717 (IL)
(72) Inventor: ADAWI, Eid, 2066717 Yokneam (IL); ROSENBERG, Avigdor, 2066717 Yokneam (IL); DEKEL, Zvi, 2066717 Yokneam (IL); MASSARWA, Fady, 2066717 Yokneam (IL)
(74) Representative: Carpmaels & Ransford LLP

(56) References cited:
- EP-A1- 3 960 073
- WO-A1-2013/192598
- WO-A1-2018/017811
- WO-A1-2018/064398
- WO-A1-2018/106569
- US-A1- 2020 198 147

## Description

### FIELD OF INVENTION

The subject matter disclosed herein relates to a system for alignment of a balloon ablation catheter in a pulmonary vein.

### BACKGROUND

The alignment of a balloon ablation catheter can be both difficult to achieve and critical to the ultimate success of the performed ablation. According to key opinion leaders in balloon ablation technologies (such as the BWI RF Heliostar, Cryoballoon, etc.), the relative orientation between the ablation balloon and the target pulmonary vein (PV) is an important parameter and considered a key predictor for generating an effective and single-shot ablation. The physicians aim for linear/axial alignment between the vein, the catheter, and the supporting sheath. This is understood to be the optimal ablation approach for using a balloon catheter. A guiding technique for alignment of the balloon ablation catheter and its sheath relative to the target pulmonary vein is considered an unmet clinical need. As such, alignment techniques to improve the alignment of the catheter and/or the ease of alignment of the catheter are needed.

EP3960073A1 describes a method which includes, using anatomical data, calculating multiple cross-sectional shapes of a lumen of an organ in a body of a patient at multiple respective positions on a medial axis of the lumen. One or more of the positions on the medial axis are identified, at which a given expandable frame of a catheter would fit the cross-sectional shapes. The one or more identified positions are presented to a user.

WO2018/106569A1 describes electrophysiology catheters for tissue ablation within a cardiac muscle, and in particular, an ablation balloon and catheter shaft that deflects to conform to a shape of a target pulmonary vein receiving ablation therapy for a cardiac arrhythmia.

WO2018/064398A1 describes systems for diagnosing and/or treating arrhythmia of a heart which make use of an array of articulation balloons to control movement of a distal portion of a catheter inside the heart.

WO2018/017811A1 describes automated systems and methods for removing hair from a body surface.

### SUMMARY

The invention is defined in appended claim 1. Embodiments of the invention are defined in the dependent claims.

### BRIEF DESCRIPTION OF THE DRAWINGS

A more detailed understanding may be had from the following description, given by way of example in conjunction with the accompanying drawings wherein:
FIG. 1 illustrates a depiction of the linear alignment according to an aspect of the present system and method;
FIG. 2 illustrates a depiction of the lack of alignment according to an aspect of the present system and method;
FIG. 3 illustrates a method of aligning to achieve the linear alignment of FIG. 1;
FIG. 4 illustrates a depiction of an image in which the axis of the vein is shown;
FIG. 5 illustrates a depiction of an image in which the axis of the balloon is shown;
FIG. 6 illustrates a depiction of an image in which the axis of the sheath is shown;
FIG. 7 illustrates an exemplary depiction of an image in which the axis of the vein and the axis of the balloon are shown;
FIG. 8 illustrates an exemplary depiction of an image in which the axis of the vein and the axis of the balloon are shown;
FIG. 9 illustrates an exemplary depiction of the automated guidance of balloon positioning in conjunction with the alignment described herein;
FIG. 10 illustrates a method of the automated guidance of balloon positioning associated with the depiction of FIG. 9; and
FIG. 11 is an example diagram of a catheter-based cardiac mapping system.

### DETAILED DESCRIPTION

The present invention offers a three-dimensional (3D) visualization approach to enable the physician to achieve alignment between the target vein, the ablating catheter, and the sheath. Using an EP mapping system (such as CARTO, for example) that enables the visualization of the catheters, the sheaths, and vein anatomy, it is possible to visualize the spatial location and orientation of each. Thus, it is possible to calculate the relationship between the orientation of the catheters, the sheaths, and vein anatomy and to quantify the alignment. This quantification of the alignment may lend itself to automation and robotic alignment.

According to one embodiment, it is possible to view the orientation vector of each of the target vein, the ablating catheter, and the sheath in the 3D space, and to provide a guide to the physicians to enable the physician to bring the catheter and/or the sheath to a desired spatial position and orientation, in accordance with the ablation strategy.

Balloon ablation catheters, or other types of multi-electrode ablation catheters, are used in atrial fibrillation (AFIB) procedures to ablate the heart tissue at the ostium of the pulmonary veins (PVs), in order to isolate any electrical activity from within the veins to reach the left atria (LA) and therefore prevent it from causing atrial fibrillation disorder of the atria. Balloon ablation catheters, or other types of multi-electrode ablation catheters, are efficient if performing the electrical isolation of the veins through performing a single-shot isolation of the whole circumference of the vein within a single of few ablations.

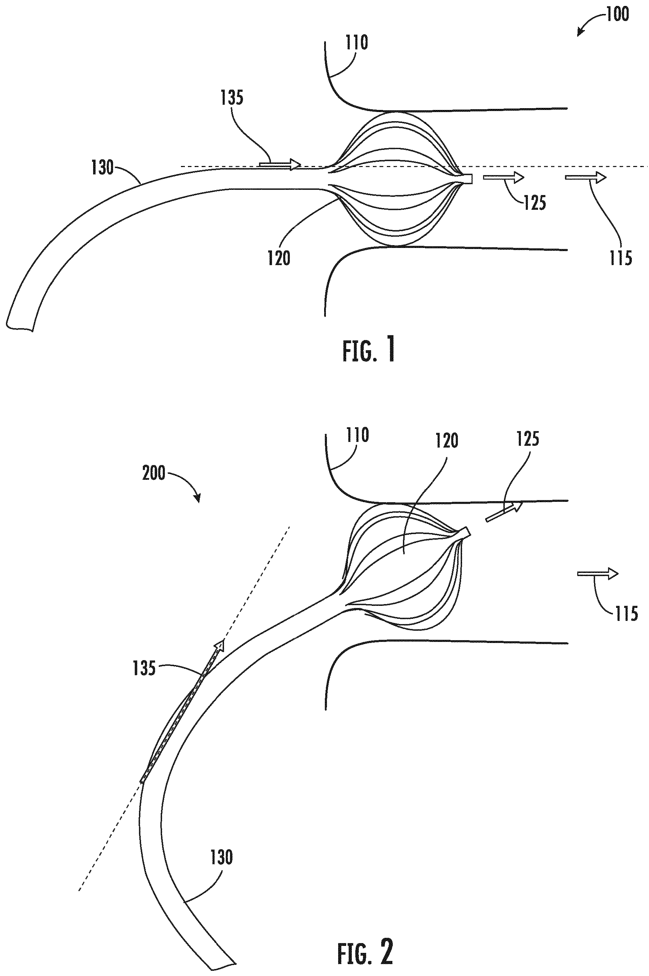

Figure 1 illustrates a depiction of the linear alignment 100 according to an aspect of the present system and method. Alignment 100 is defined in that ostium 110 is provided with a balloon 120 included therein to perform the ablation. Attached to the balloon 120 is a sheath 130.

Each of ostium 110, balloon 120 and sheath 130 has an associated axis. The axis associated with ostium 110 is illustrated as an axis indicated by the labeled ostium axis arrow 115. The axis associated with balloon 120 is illustrated as an axis indicated by the labeled balloon axis arrow 125. The axis associated with sheath 130 is illustrated as an axis indicated by the labeled sheath axis arrow 135. There is proximate alignment of the axes indicated by ostium axis arrow 115, balloon axis arrow 125 and sheath axis arrow 135.

Figure 2 illustrates a depiction of the lack of alignment 200 according to an aspect of the present system and method. The lack of alignment 200 is defined in that ostium 110 is provided with a balloon 120 included therein to perform the ablation. The balloon 120 passes through the sheath 130.

Each of ostium 110, balloon 120 and sheath 130 again has an associated axis. The axis associated with ostium 110 is illustrated as an axis indicated by the labeled ostium axis arrow 115. The axis associated with balloon 120 is illustrated as an axis indicated by the labeled balloon axis arrow 125. The axis associated with sheath 130 is illustrated as an axis indicated by the labeled sheath axis arrow 135.

As may be seen by comparing ostium axis arrow 115, balloon axis arrow 125 and sheath axis arrow 135 in Figure 2, there is substantial misalignment between the axis of the vein, balloon, and sheath.

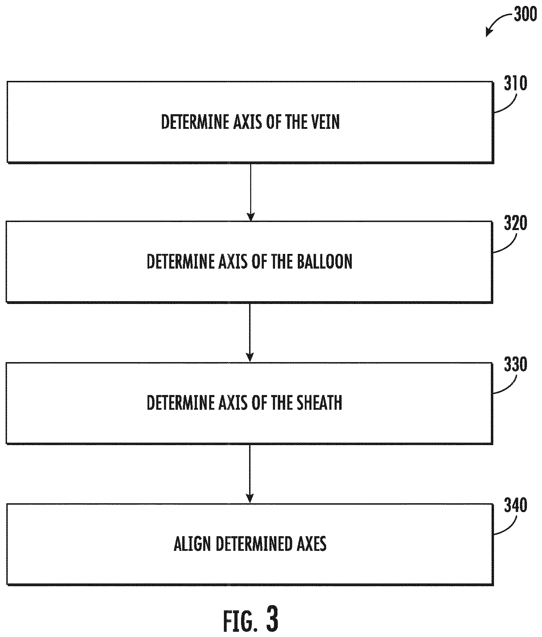

Figure 3 illustrates a method 300 for aligning to achieve the linear alignment of FIG. 1. Method 300 includes determining the axis of the vein at step 310, determining the axis of the balloon at step 320 and determining the axis of the sheath at step 330. While method 300 includes determining the axis of all three elements in steps 310-330, one of ordinary skill in the art will appreciate that this is only an embodiment, and embodiments where the vein and the balloon axes are determined along with other combinations of the three elements.

Method 300 further includes aligning the determined axes to achieve the linear alignment of FIG. 1 at step 340.

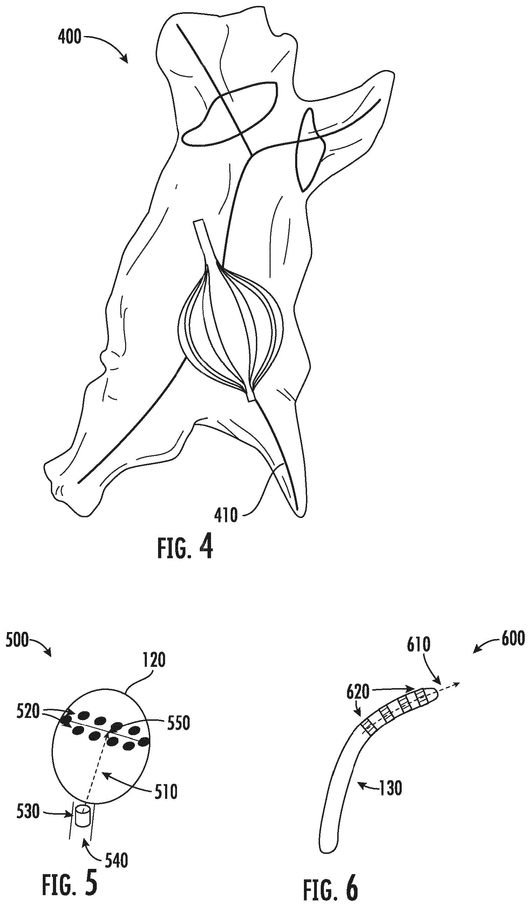

In step 310, the axis of the vein is determined. In order to perform this step of method 300, the skeleton of the vein may be generated to provide the axis of the vein in cross section. FIG. 4 illustrates a depiction of an image 400 in which the axis of the vein 410 is shown. Axis 410 is determined by as described below and displayed in image 400. One of the many possible methods for finding the axis of the vein is described. The center point of the vein is determined using a virtual slice-cut through the shell of the map at the antrum to the vein by calculating a best-fit circle of the shell at the cut point and finding its geometrical center. A second point is determined by moving into the vein to perform a second virtual slice-cut, in which its center is found in a similar manner to the first cut. A third point is determined using a next slice and performing the same slice-cut steps described above. These slice-cut centers may be repetitively performed. The centers may be connected by a best fit line in order to identify the direction of the vein.

In step 320, the axis of the balloon is determined. The axis of the balloon may be determined by finding the vector connecting between the magnetic location sensor at the shaft of the balloon and the center of the best-fit circle of the locations of the balloon electrodes, to provide the axis vector. FIG. 5 illustrates a depiction of an image 500 in which the axis of the balloon 510 is shown. Axis 510 is determined by determining the center of a best fit circle 550 of the location of electrodes 520 of balloon 120 and drawing a line from the magnetic sensor 530 in the shaft 540 of balloon 120 to the center 550. Axis 510, as shown, is the vector connecting the center of magnetic sensor 530 and the determined center 550. Axis 510 is displayed in image 500. As would be understood by those possessing an ordinary skill in the pertinent arts, there are known algorithms for finding the best-fit circle from a set of points. For example, an algorithm based on the minimizing the sum of the squares of the distances between the locations of the electrodes to the closest points on the circle may be utilized. Alternatively, the algebraic and/or geometric distance to the points may be minimized. A combination of techniques may also be used with weighting utilized on top of the combination of algorithms.

In step 330, the axis of the sheath may be determined. The axis of the sheath may be determined using a magnetic sensor(s) or rings to visualize the sheath and calculate the vector of the sheath. FIG. 6 illustrates a depiction of an image 600 in which the axis 610 of the sheath 130 is shown. Axis 610 is determined as the direction vector passing between the locations 620 of the distal ring electrodes placed on the sheath 130. The ring electrodes placement at locations 620 along the sheath 130 may be in any location that allows a direction vector of the rings to be determined. One example shown in FIG. 6 is to use concentric evenly spaced rings on sheath 130. Alternatively, or additionally, a direction of a magnetic sensor(s) mounted around the distal edge of the sheath 130 may be used. Axis 610 is displayed in image 600.

The locations 620 and directions of the ring electrodes (magnetic sensors) may be performed using the location of a navigation system, such as CARTO, for example. For example, for ring electrodes 620, a series of currents may be transmitted through the ring electrodes. A series of patch-electrodes on a patient's body may be used to read the received currents. Once read the locations may be calculated using distributed current ratios.

Alternatively, or additionally, magnetic locations may be utilized. By transmitting magnetic fields from static coils, such as from under a patient's bed, for example, the received magnetic fields may by measured using magnetic sensors. The magnetic sensors generally may be coils, and may be constructed of either printed leads on a flexible printed circuit substrate or wire-wound coils. The vectors may then be displayed on the display of the navigation system.

In order to align the determined axes at step 340 of method 300, an estimate of any misalignment maybe provided. For example, the distance between the balloon axis and the vein axis may be estimated. The angle between any one of the axes of the balloon, the axis of the vein and the axis of the sheath may be estimated and provided in a display. The display provided may include the vectors and/or the distance between the vectors.

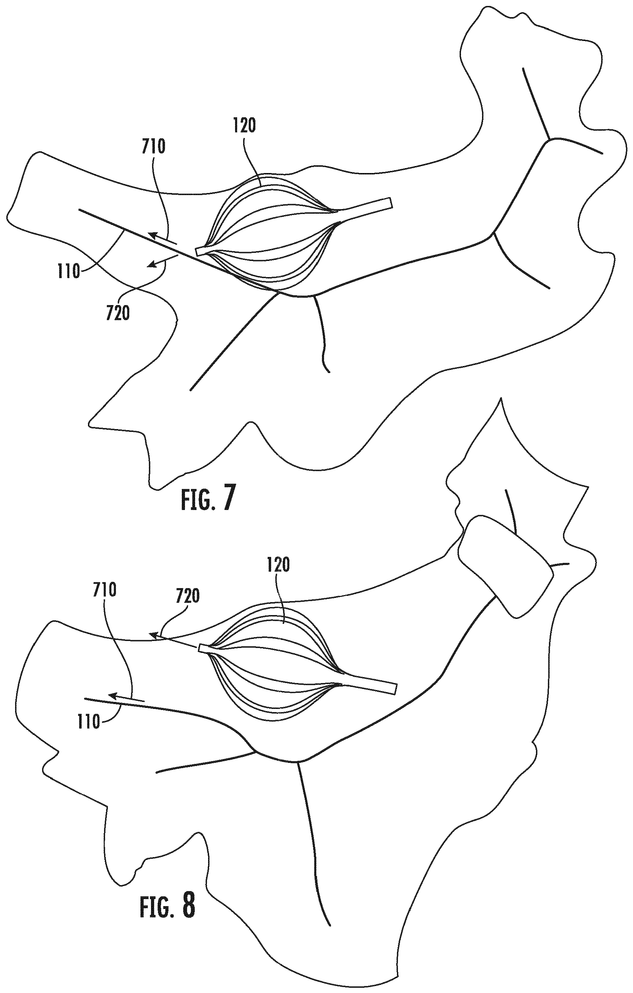

FIG. 7 illustrates a depiction of an image in which the axis of the vein 710 and the axis of the balloon 720 are shown. The balloon 120 and vein 110 are also shown in the image. The angle between the axis of the vein 710 and the axis of the balloon 720 is provided. The arrow 710 is the direction vector of the vein axis and the arrow 720 is the direction vector of the balloon axis.

FIG. 8 illustrates a depiction of an image in which the axis of the vein 710 and the axis of the balloon 720 are aligned to be in same direction. There is a shift/displacement between the balloon vector 720 and the vein axis 110. This shift/displacement may require correction in order to achieve balloon-to-vein alignment. Additionally, the balloon 120 and vein 110 are also shown in the image.

As may be observed from the images of FIGs. 7-8, the angle between the balloon direction vector and the vein direction vector and the shift/displacement between the vectors in each figure displays the vectors and/or the distance between the vectors until the distance between the vectors is zero indicating alignment between the axis of the balloon 720 and the axis of the vein 710. While FIGs. 7-8 depict the vein and balloon, similar configurations may be included with the sheath, and any combination of the vein, balloon and sheath.

As discussed herein, navigating and positioning a catheter, such as a balloon catheter into the vein often requires complicated and potentially time-consuming maneuvering and experience. As described, the present system and method for alignment of the balloon ablation catheter in the pulmonary vein may be expanded to include automated guidance of balloon position. This navigation may be performed by a deflectable sheath and by the deflection of the balloon. Such navigation may include forward and backward movement of the sheath and of the balloon, deflection of the sheath and of the balloon, and rotation of the sheath and of the balloon.

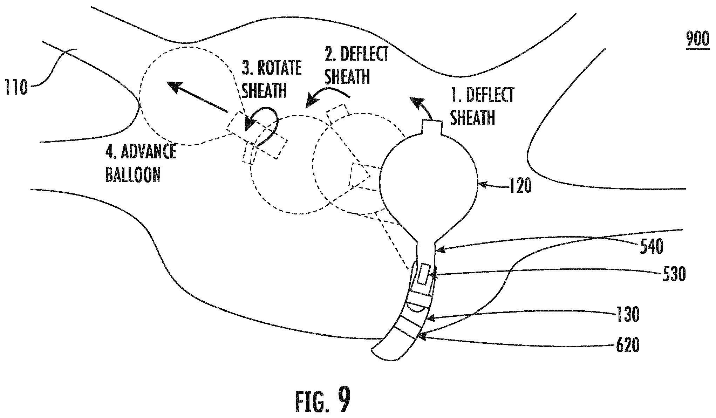

FIG. 9 illustrates an exemplary depiction 900 of the automated guidance of balloon positioning in conjunction with the alignment described herein. Depiction 900 includes the balloon 120 including a deflectable balloon shaft 540, and a balloon catheter magnetic sensor 530 as described herein. Depiction 900 further includes a sheath 130 with a sheath locatable sensor 620 as described herein.

Sheath 130 including magnetic sensors 620 and/or locatable electrodes on the front-end, a balloon 120 including a magnetic sensor 530 and a navigation system, such as CARTO^{®} 3, allows navigation by discerning the rotation, deflection and orientation of sheath 130 and balloon 120 and the relationship of each with respect to vein 110. The direction of sheath 130 and balloon 120 in order to occupy vein 110 may be realized via every twist/movement of the deflection knob of the sheath and of the balloon catheter.

A pre-acquired, or concurrently acquired, 3D map of the chamber of the heart, and the current location, rotation, deflection state and orientation of sheath 130 and balloon 120 enables a prediction of the next position of balloon 120, relative to the 3D map, as a result of movement of sheath 130 and balloon 120. Additionally, or in conjunction, the optimal trajectory for placing balloon 120 into the correct position in vein 110 may be calculated.

By calculating the optimal trajectory of balloon 120 to arrive in vein 110, a step-by-step direction set for the operator to operate sheath 130 and balloon 120 advancement including rotation and deflection movements may be provided. This may allow the balloon 120 to optimally and quickly be positioned into vein 110. This same positioning algorithm and sensors may be also used for a robotic system, for automated guidance of sheath 130 and balloon 120 into vein 110.

In the exemplary alignment illustrated in FIG. 9, the alignment process attempts to move balloon 120 from the first initial position through a series of intermediate positions to the in-place for procedure position in vein 110. In the exemplary illustration, the balloon 120/sheath 130 are manipulated through a series of positions to arrive at vein 110. Specifically, from an initial position of balloon 120, the sheath 130 is deflected to arrive at a second intermediate position. From there the sheath 130 is deflected again to arrive at a third intermediate position. Then the sheath 130 is rotated to arrive at a fourth intermediate position. Finally, in a final alignment step the balloon 120 is advanced into the vein 110. While the exemplary maneuvers of the balloon 120 and sheath 130 in propagating to vein 110 are specific, they are provided as exemplary maneuvers. Any number of maneuvers may be required to reach vein 110. Similarly, the order of the specific maneuvers may be varied. As one would understand any combination, or order of maneuvers may be used.

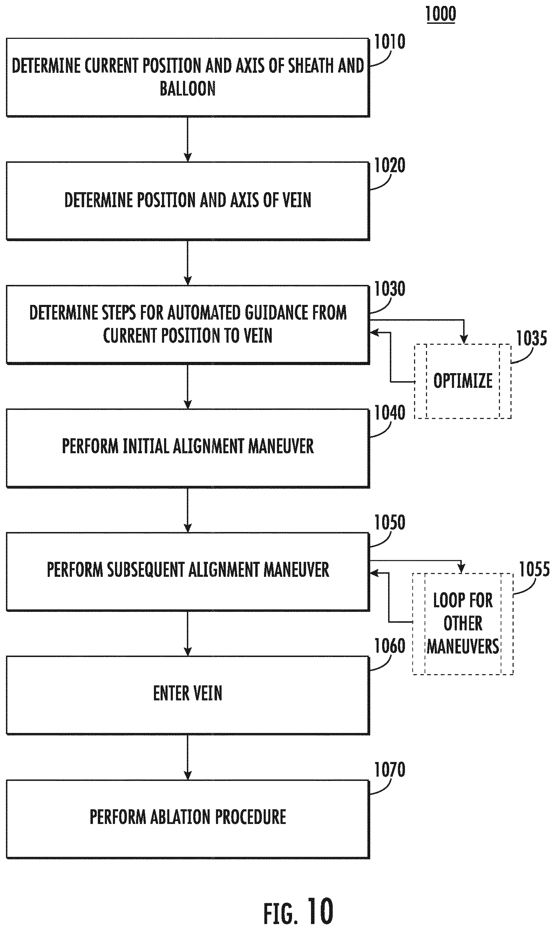

FIG. 10 illustrates a method 1000 of the automated guidance of balloon positioning associated with the depiction of FIG. 9. As illustrated in FIG. 10, at step 1010 method 1000 includes determining the current position and axis of at least one of the sheath 130 and balloon 120, This determination may be performed as described herein. Method 1000, at step 1020, includes determining the position and axis of vein 110. This determination may be performed as described herein. Method 1000, at step 1030, includes determining the steps for automated guidance from the current position of the balloon 120 to the vein 110. At step 1035, method includes the optional optimization of the steps for automated guidance in step 1030. This optimization 1035 may include minimizing the number of maneuvers required. Alternatively, the optimization at step 1035 may include a weighting of the kind of maneuvers, such as a preference for deflection for example. Similarly, the optimization may include parameters that account for the time duration of each maneuver and the optimization at step 1035 minimizes the time required to reach vein 110, for example.

Once the steps are determined at step 1030, and optimized at step 1035 if desired, method 1000 includes performing an initial alignment maneuver at step 1040. At step 1050, method 1000 includes performing a subsequent alignment maneuver. This subsequent maneuver may be looped at step 1055 by performing other maneuvers in sequence to guide the balloon 120 into the vein 110 as determined by the determined steps of step 1030. At step 1060, once the maneuvers are performed, the vein 110 may be entered. Once configured in the vein 110 at step 1060, the ablation procedure may be performed at step 1070.

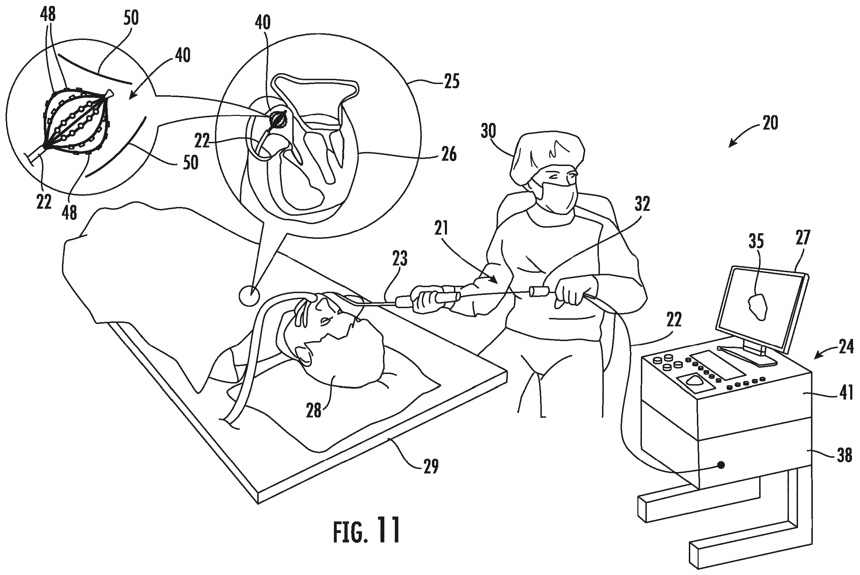

FIG. 11 is an illustration of a catheter-based cardiac mapping system 20 comprising an ultrasound basket catheter 40, in accordance with an embodiment of the present invention. It will be understood that although a basket shape is disclosed throughout, any shape catheter comprising multiple transducers may be used to implement the embodiments disclosed herein. System 20 comprises a catheter 21, having a shaft 22 that may be navigated by a physician 30 into a heart 26 of a patient 28 lying on a table 29. As shown in FIG. 11, physician 30 may insert shaft 22 through a sheath 23, while manipulating the distal end of shaft 22 using a manipulator 32 near the proximal end of the catheter and/or deflection from the sheath 23. As shown in an inset 25, basket catheter 40 may be fitted at the distal end of shaft 22. Basket catheter 40 may be inserted through sheath 23 in a collapsed state and may be then expanded within heart 26.

In an embodiment, basket catheter 40 may be configured to perform spatial mapping of a cardiac chamber of heart 26 by transmitting wide and narrow echo signals and receiving wide and narrow echo signals that were reflected from cardiac chamber surfaces 50, and which information may be used to form a map of the heart chamber and veins to which the catheter is intended to be navigated. Alternatively, the map of the chamber may be built using the locations of the electrodes of the basket or balloon catheter, through use of a 3D mapping system, such as CARTO^{®}. An inset 45 shows basket catheter 40 in an enlarged view, inside a cardiac chamber of heart 26.

The proximal end of catheter 21 may be connected to a console 24. Console 24 may include a processor 41, such as a general-purpose computer, with suitable front end and interface circuits 38 for transmitting and receiving signals to and from catheter 21, as well as for controlling the other components of system 20. In some embodiments, processor 41 may be further configured to receive multiple dual frequency (e.g., wide and narrow) echo signals and to calculate a map of a surface of a cardiac chamber from the echo signals. In an embodiment, the surface of the surrounding anatomy may be presented to physician 30 on a display 27, e.g., in a graphical form of a mesh diagram 35.

As noted above, processor 41 may include a general-purpose computer, which may be programmed in software to carry out the functions described herein. The software may be downloaded to the computer in electronic form, over a network, for example, or it may, alternatively or additionally, be provided and/or stored on non-transitory tangible media, such as magnetic, optical, or electronic memory. The example configuration shown in FIG. 11 is chosen purely for the sake of conceptual clarity. The disclosed techniques may similarly be applied using other system components and settings. Additionally, system 20 may include additional components, such as ones for electrophysiological mapping and/or ablation. Although the pictured embodiment relates specifically to the use of an ultrasound basket catheter for cardiac mapping, the elements of system 20 and the methods described herein may alternatively be applied in ultrasound mapping using catheters having other multi-arm geometries, or non-ultrasound 3D mapping using basket, balloon or other catheters types.

Currently, physicians navigate the catheters (based on Fluoroscopy, ICE, or a mapping system) and independently (without guidance or quantitative tools) try to understand the relative orientation between the entities and to align the moving parts. There is no quantification of the alignment degree.

The invention is based on the visualization capabilities of the system. The invention is aimed for linear alignment between the entities. The configuration could be changed according to the positioning and ablation strategy. This invention could be extended to other catheter types (e.g., focal catheters), if there is need for specific alignment during ablation.

While the present description details correcting linear alignment of the balloon, ostium, and sheath, this concepts in this description may be used to assist physicians in achieving non-linear, slanted, lateral ablation, or any other combination of balloon-vein-sheath configurations for an ablation approach, as needed. While generally the present examples provide for linear alignment as this is often preferred in ablations, other combinations of balloon-vein-sheath configurations, including non-linear, slanted, lateral ablations, etc., may be configured.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor.

## Claims

1. A system for achieving linear alignment between elements in a robotic surgical procedure on a patient, comprising:
an ablation catheter comprising a shaft (540), a magnetic location sensor (530) on the shaft, and a plurality of electrodes (520);
a processor (41) configured to:
determine (310) an axis (410) of a vein of the patient by performing a plurality of virtual slice-cuts of a shell of a map of the vein, calculating best-fit circles of the shell at the slice-cuts, determining geometrical centers of the best-fit circles, and connecting the geometrical centers by a best-fit line to identify the axis of the vein;
determine (320) an axis (510) of the ablation catheter by finding a vector connecting the magnetic location sensor and a center (550) of a best-fit circle of locations of the plurality of electrodes; and
align (340) the determined axis of the vein and the determined axis of the ablation catheter by providing control of the ablation catheter for automatic alignment of the determined axis of the vein and the determined axis of the ablation catheter.

2. The system of claim 1, the processor further configured to determine (330) the axis (610) of a sheath (130) utilized in the procedure and align the determined axis of the sheath with the aligned axis of the vein and the aligned axis of the ablation catheter.

3. The system of claim 1, wherein the ablation catheter comprises a balloon catheter (120).

## Patentansprüche

1. System zum Erreichen einer linearen Ausrichtung zwischen Elementen bei einem robotischen chirurgischen Eingriff an einem Patienten, umfassend:
einen Ablationskatheter, umfassend einen Schaft (540), einen magnetischen Ortungssensor (530) auf dem Schaft und eine Vielzahl von Elektroden (520);
einen Prozessor (41), der konfiguriert ist zum:
Bestimmen (310) einer Achse (410) einer Vene des Patienten durch ein Durchführen einer Vielzahl von virtuellen Scheibenschnitten einer Hülle einer Karte der Vene, Berechnen von am besten passenden Kreisen der Hülle an den Scheibenschnitten, Bestimmen von geometrischen Mittelpunkten der am besten passenden Kreise und Verbinden der geometrischen Mittelpunkte durch eine am besten passende Linie, um die Achse der Vene zu identifizieren;
Bestimmen (320) einer Achse (510) des Ablationskatheters durch ein Finden eines Vektors, der den magnetischen Ortungssensor und einen Mittelpunkt (550) eines am besten passenden Kreises von Orten der Vielzahl von Elektroden verbindet; und
Ausrichten (340) der bestimmten Achse der Vene und der bestimmten Achse des Ablationskatheters durch ein Bereitstellen einer Steuerung des Ablationskatheters für die automatische Ausrichtung der bestimmten Achse der Vene und der bestimmten Achse des Ablationskatheters.

2. System nach Anspruch 1, wobei der Prozessor ferner konfiguriert ist, um die Achse (610) einer Hülle (130) zu bestimmen (330), die bei dem Eingriff verwendet wird, und die bestimmte Achse der Hülle mit der ausgerichteten Achse der Vene und der ausgerichteten Achse des Ablationskatheters auszurichten.

3. System nach Anspruch 1, wobei der Ablationskatheter einen Ballonkatheter (120) umfasst.

## Revendications

1. Système permettant d'obtenir un alignement linéaire entre des éléments lors d'une procédure chirurgicale robotisée sur un patient, comprenant :
un cathéter d'ablation comprenant une tige (540), un capteur de localisation magnétique (530) sur la tige, et une pluralité d'électrodes (520) ;
un processeur (41) configuré pour :
déterminer (310) un axe (410) d'une veine du patient en effectuant une pluralité de coupes virtuelles d'une enveloppe d'une représentation de la veine, en calculant des cercles d'ajustement optimal de l'enveloppe au niveau des coupes, en déterminant des centres géométriques des cercles d'ajustement optimal et en reliant les centres géométriques par une ligne d'ajustement optimal afin d'identifier l'axe de la veine ;
déterminer (320) un axe (510) du cathéter d'ablation en trouvant un vecteur reliant le capteur de localisation magnétique et un centre (550) d'un cercle de localisations d'ajustement optimal de la pluralité d'électrodes ; et
aligner (340) l'axe déterminé de la veine et l'axe déterminé du cathéter d'ablation par la fourniture de commande du cathéter d'ablation pour un alignement automatique de l'axe déterminé de la veine et de l'axe déterminé du cathéter d'ablation.

2. Système selon la revendication 1, le processeur étant en outre configuré pour déterminer (330) l'axe (610) d'une gaine (130) utilisée dans la procédure et aligner l'axe déterminé de la gaine avec l'axe aligné de la veine et l'axe aligné du cathéter d'ablation.

3. Système selon la revendication 1, dans lequel le cathéter d'ablation est un cathéter à ballonnet (120).
